Europäisches Patentamt

European Patent Office

Office européen des brevets

(11) Publication number: **0 519 415 A1**

## EUROPEAN PATENT APPLICATION

(21) Application number: **92110202.6**

(22) Date of filing: **17.06.92**

(51) Int. Cl.5: **A61N 5/02**

(30) Priority: **19.06.91 IT FI910152**

(43) Date of publication of application:
**23.12.92 Bulletin 92/52**

(84) Designated Contracting States:
**BE DE DK ES FR GB IT NL SE**

(71) Applicant: **SMA SEGNALAMENTO MARITTIMO ED AEREO S.p.A.**
**Via del Ferrone, 5**
**I-50124 Firenze(IT)**

(72) Inventor: **Borrani, Antonello**
**Via Baldesi, 6**
**I-50131 Florence(IT)**
Inventor: **Benini, Daniele**
**Via Villani, 34**
**I-50124 Florence(IT)**

(74) Representative: **Gervasi, Gemma et al**
**NOTARBARTOLO & GERVASI Srl Viale**
**Bianca Maria 33**
**I-20122 Milan(IT)**

(54) Microwave apparatus for clinical hyperthermia in endogenous thermotherapy.

(57) A microwave apparatus for clinical hyperthermia in endogenous thermotherapy comprises a radiofrequency electromagnetic energy generator (1), an applicator (3) comprising a ridged conical-horn antenna (31) for administration of the electromagnetic energy, a skin temperature sensor (4) comprising insulating and preamplificating means (41), a bag (32) waterproof connected to the radiant opening of the antenna (31) and made of flexible dielectric material transparent to radiofrequency signals up to 500MHz and containing a thermoregulatory fluid with forced circulation and adjustable temperature and pressure; a control unit (6) comprising a microprocessor, an operator panel (82) and a personal computer (9), if any, for setting, control, processing and display of the processing data and driving of the peripheral elements.

TABLE 1

Fig. 1

The present invention refers to a microwave apparatus for clinical hyperthermia in endogenous thermotherapy.

In the orthopedic-traumatological and rheumatological field the use of endogenous thermotherapy for curing the affections by heating the affected body parts is known. Known apparatuses employed for this purpose are those for diathermy and radar therapy.

Various difficulties are due however to the use of these known apparatuses. Those for diathermy have poor heat focussing on the affected part, high absorption by the adipose layers with resulting overheating and high electromagnetic pollution of the environment. Those for radar therapy in the S band, e.g. 2450MHz, have poor penetration of radiation and high absorption by the surface tissues with serious risk of burning.

Apparatuses for radar therapy working in UHF band, e.g. 434MHz, are also known which comprise emitting means placed at a certain distance from the skin: the relevant treatment provides only for the control of the power and of the duration of emission. In these known machines in addition a not negligible part of energy is lost by reflection from the air-skin surface and therefore effective control of power actually absorbed is not possible.

Nevertheless, because of the high surface absorption, the thermoreceptors are highly energized and hence power control by the operator based on subjective patient's sense of heat causes very often an under-dosage condition.

Finally, there are known apparatuses for electromagnetic oncological hyperthermia applied in association with chemotherapy and/or radiotherapy. Said apparatuses are characterized in that they are very complicated, requiring in particular the continuous taking of the temperature inside the tissues, even in several points.

A purpose of the present invention is to provide a microwave apparatus for clinical hyperthermia in endogenous thermotherapy which would eliminate said shortcomings of known machines and in particular allow control of the treatment parameters while keeping under control the patient's skin temperature.

This result is achieved in accordance with the present invention by providing an apparatus comprising:

- a radiofrequency electromagnetic energy generator,
- means for administration of the electromagnetic energy,
- means for control and regulation of the patient's skin temperature, and
- means comprising a microprocessor for acquisition and processing of treatment data and for driving the peripheral elements.

Advantageously in accordance with the present invention said energy administration means comprise an applicator with a 'ridged conical horn' antenna. Said temperature control and regulation means comprise respectively a skin temperature sensor and a bag made of flexible dielectric material transparent to radiofrequencies up to 500MHz and containing a thermoregulatory fluid, e.g. distilled water, with forced circulation and adjustable temperature and pressure, said bag being fixed in a waterproof manner to the antenna radiant opening and said applicator and said bag being supported by an arm movable in three spatial directions and being turnable in any direction.

The advantages achieved with the present invention consist essentially of the fact that there is a high electromagnetic matching between the means (water and body tissues) in which the electromagnetic radiation propagates: the power delivered is therefore completely transferred inside the body with no loss by reflection; that consequently, for given power delivered there is more penetration of the radiation or less power is required to bring the tissues to the same temperatures; that it is also possible a better focusing of the radiation; that the control of skin temperature done by changing water temperature, allows control of the degree of penetration of radiation and hence the depth of highest temperature in the tissues; that the risk of burns is practically eliminated; that the absence of reflections on the skin surface reduces electromagnetic pollution of the environment; that distribution of the therapeutic temperatures is reached in a broader region and its value can be controlled; that by means of the flexible bag a complete mechanical adaptation between the applicator surface and the part subjected to treatment is reached; and that consequently the primary fields of application are rheumatic polymyalgia, periarthritis of the shoulder and hip, lumbago, tenosynovitis, tendonitis, bursitis, carpal and tarsal channel syndrome, articular gout in chronic form, arthrosis, inflammatory and noninflammatory myopathy (trauma), brachialgia, dorsalgia, lumbar and lumbosacral pain, and traumatology in general.

The innovative principles of the present invention and its advantages as compared with the known art will be better understood by a skilled person through the following description, done as nonlimiting example, and the enclosed drawings, where:

FIG. 1 shows a block diagram of a microwave apparatus for clinical hyperthermia in the endogenous thermotherapy in accordance with the present invention,

FIG. 2 shows a schematic overall view of an apparatus in accordance with the block diagram of FIG. 1,

FIG. 3 shows a thermal profile of tissues subjected to treatment without the surface temperature control, and

FIG. 4 shows the thermal profile of similar tissues subjected to treatment with an apparatus in accordance with the present invention with the surface temperature control.

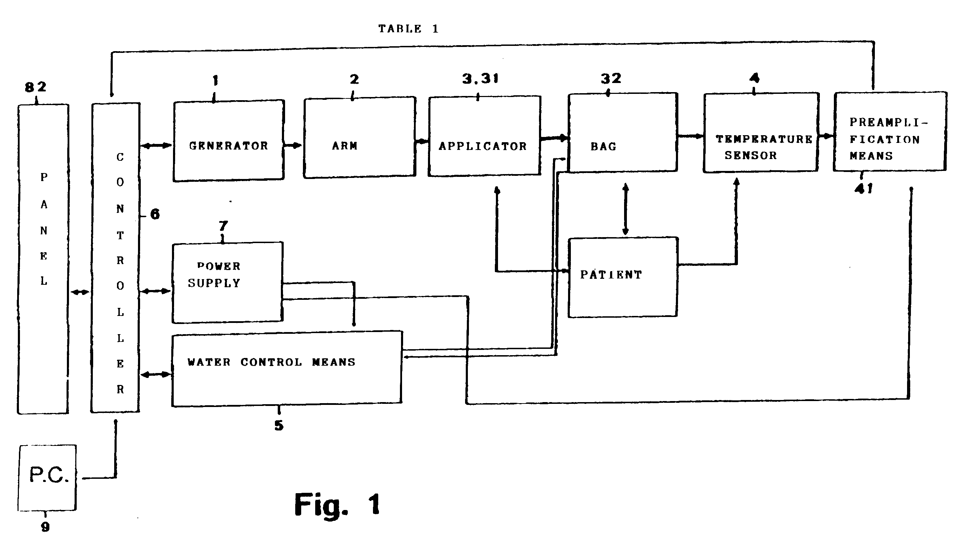

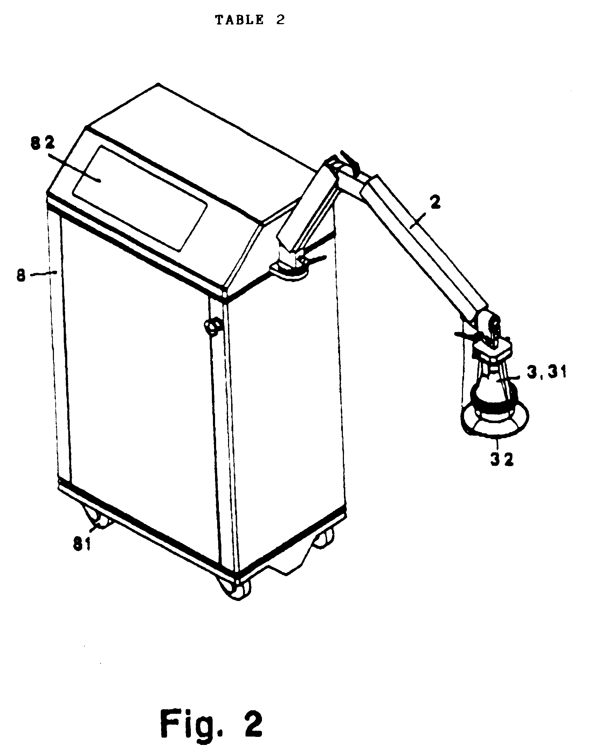

Reduced to its essential structure and with reference to FIGS. 1 and 2 an apparatus for hypothermal microwave endogenous therapy in accordance with the present invention comprises:

- a 434MHz radiofrequency electromagnetic energy generator 1 with a continuously controlled output power from 0W to 100W,
- an arm 2 for support and positioning of an applicator 3 with a ridged conical horn antenna 31 and a sealed bag 32 of flexible dielectric material transparent to radiofrequencies up to 500MHz and containing distilled water under forced circulation, means being provided for hydraulic and electromagnetic interconnections as well as jointing and self-balancing of the arm 2 to ensure the degrees of freedom necessary and to allow blocking of the applicator 3 in the selected position without need of fixing it to the patient,
- a skin temperature sensor 4, e.g. a thermocouple of the copper-constantan T type, equipped with an insulation and preamplification device 41,
- means 5 for circulation of water and pressure and temperature control thereof in the bag 32 in the range 37°C-42°C,
- controller 6 of the equipment with a microprocessor for processing of treatment data,
- a power supply 7 to supply all the voltages and currents necessary for the working of the various units while assuring insulation from the external power line.
- a boxed frame 8 mounted on pivoting wheels 81 containing the functional units 1,5,6,7 of the apparatus with an operator panel 82 for setting, control and display of treatment data.

In a preferred embodiment, control means 6 comprise data support means such as a read only memory (ROM) in which the characteristic thermal profiles of one or more body portions are memorised.

In another preferred embodiment an apparatus in accordance with the present invention is equipped with a personal computer 9 which constitutes the interface with the operator and allows increasing the processing efficiency, filing of treatment data for each patient and optionally providing for memorization of characteristic thermal profiles into the above data support means.

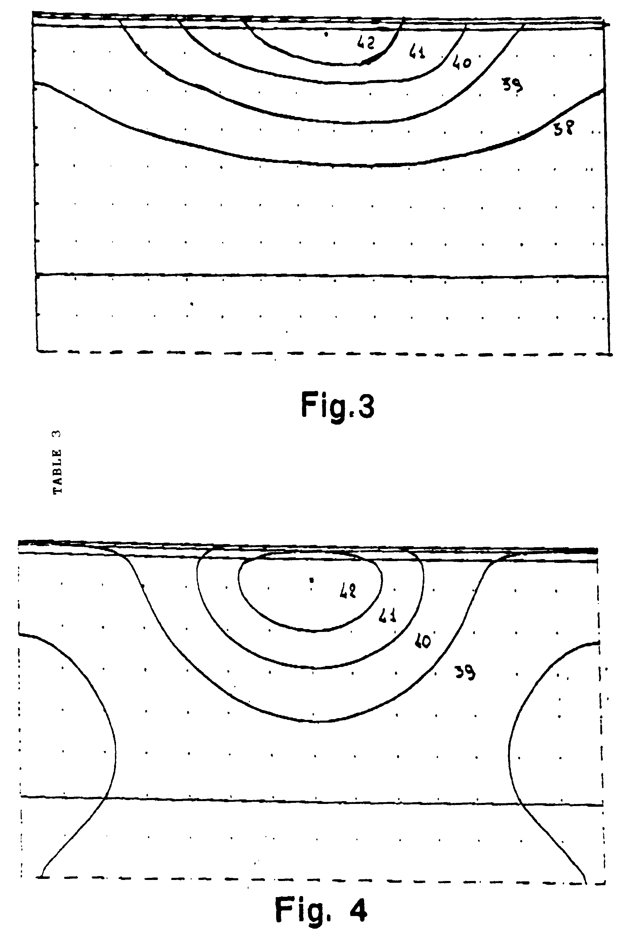

Working is as follows. The operator sets on the panel 82 or by means of the personal computer 9 the maximum power and duration of the application. Then, on the basis of the temperature distribution to be obtained in depth, the temperatures of the skin and of the bag 32 are set, again on the panel 82 or by means of the personal computer 9. Indeed, as shown in FIGS. 3,4 the thermal profile of the tissues subjected to treatment depends heavily if the bag 32 is used (FIG. 4) or not (FIG. 3) and is also a function of the temperature of the water contained therein.

Then the arm 2 is moved with the applicator 3 so that the bag 32 adheres to the affected skin surface of the patient; in particular the flexibility of the bag 32 and the capability of control of the pressure of the water contained therein allows a complete mechanical adaptation between the applicator 3 and the skin surface.

During treatment the data set on the panel 82 are processed, (together with the skin temperature data coming through the thermometric unit 41 from the sensor 4 by the microprocessor belonging to the control means 6 and in accordance with a specific algorithm which allows for the typical thermal profiles in the tissues. By means of said processing the treatment parameters are controlled.

It is clear that when a personal computer 9 is used, it provides said processing.

Skin temperature is continuously checked by the sensor 4 and delivery of power is consequently controlled by the controller 6 to maintain the preselected temperature.

Among the functions performed through the panel 82, in addition to those mentioned of setting the treatment parameters and the usual ones of turning the apparatus on or off or starting and stopping treatment and indication of trouble if any, there are those of indication of congruence of the data set with predetermined limit values and indication of the power delivered and reflected. In this manner the operator can intervene appropriately to correct or change the setting and or position more effectively the applicator 3.

The assumed values of the treatment parameters with the apparatus which is the object of the present invention are for example:

- output frequency: 434MHz,
- power delivered: 0-100W,
- skin temperature: 38-40°C;
- temperature of thermoregulatory fluid: 37-42°C.

The microprocessor used is typically but not exclusively of the 8 bit type and is equipped with a 32 kbyte memory.

The apparatus proposed is realized in accordance with applicable safety standards for electromedical equipment and in particular for microwave therapy equipment.

It is clear that in practice the execution details can vary in equivalent manner as to form, dimensions, arrangement of the elements and nature of the materials employed without thereby going beyond the solution idea adopted and therefore remaining within the protective field provided by the present invention.

**Claims**

1.  Microwave apparatus for clinical hyperthermia in endogenous thermotherapy characterized in that it comprises:
    - a radiofrequency electromagnetic energy generator (1),
    - electromagnetic energy administration means (3,31),
    - patient's skin temperature control and regulation means (4,41,32), and
    - means (6,82,9) including a microprocessor for treatment data acquisition and processing and for driving the peripheral elements.

2.  Apparatus in accordance with claim 1 characterized in that the output power of said generator (1) is continuously adjustable from 0W to 100W and in that its output frequency is between 3 and 300MHz or 300 and 3000MHz.

3.  Apparatus in accordance with claim 1 characterized in that said means of energy administration include an applicator (3) with a ridged conical horn antenna (31).

4.  Apparatus in accordance with claim 1 characterized in that said patient's skin temperature control and regulation means include a skin temperature sensor (4) equipped with an insulation and preamplification device (41) and a bag (32) of flexible dielectric material transparent to radiofrequencies up to 500Mhz and containing a thermoregulatory fluid, e.g. distilled water with forced circulation and controlled temperature and pressure, said bag (32) being waterproof fixed to the radiant opening of the antenna (31).

5.  Apparatus in accordance with claim 1 characterized in that said treatment data acquisition and processing means and peripheral element driving means include:
    - a controller (6) with a microprocessor for treatment data processing, and
    - a panel (82) designed for the operator for treatment data setting, control and display.

6.  Apparatus in accordance with claims 1 and 5 characterized in that for said treatment data processing a specific algorithm is provided which allows for typical thermal profiles in the tissues and regulates treatment parameter and in particular delivered power.

7.  Apparatus in accordance with claims 1 and 5 characterized in that it includes data support means such as a read only memory (ROM) in which characteristic thermal profiles of one or more body portions are memorised.

8.  Apparatus in accordance with claim 1 characterized in that said means of acquisition and processing of treatment data and for piloting of the peripheral elements include a personal computer (9).

9.  Apparatus in accordance with claim 3 characterized in that said applicator (3) is mounted on the free end of an arm (2), means being provided for hydraulic and electromagnetic interconnection as well as jointing and self-balancing of the arm (2) to ensure the necessary degrees of liberty and to allow blocking of the applicator (3) in the selected position without need to fix it to the patient.

10. Apparatus in accordance with claims 1 and 4 characterized in that it includes means (5) for circulation and control of the pressure and the temperature of the water contained in the bag (32) in the range 37-42°C.

11. Apparatus in accordance with claims 1 and 8 characterized in that said generator power (1), said means (6,82) for treatment data acquisition and processing and for piloting of the peripheral elements and said means (5) of circulation and control of water pressure and temperature are contained in a boxed frame (8) mounted on pivoting wheels (81).

TABLE 1

Fig. 1

EP 0 519 415 A1

TABLE 2

# Fig. 2

Fig.3

TABLE 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-8 803 823 (BSD MEDICAL CORPORATION)<br>* page 5, line 29 - page 6, line 32 *<br>* page 13, line 28 - line 31 *<br>* page 20, Table 1 *<br>* page 23, line 3 - line 17 * | 1-3,5-8 | A61N5/02 |
| Y | * page 14, line 26 - page 15, line 19 * | 4 | |
| X | US-A-4 884 580 (KIKUCHI ET AL) | 1,2,10 | |
| Y | * column 2, line 64 - column 5, line 4 * | 4 | |
| X | EP-A-0 207 729 (BSD MEDICAL CORPORATION)<br>* column 6, line 20 - column 7, line 24 *<br>* column 15, line 30 - line 39 * | 1,2,5,6 | |
| A | US-A-4 891 483 (KIKUCHI ET AL.)<br>* column 5, line 13 - line 68 *<br>* column 7, line 13 - line 63 * | 9-11 | |
| A | DE-A-2 151 889 (ROBERT BOSCH ELEKTRONIK GMBH)<br>* the whole document * | 11 | |

TECHNICAL FIELDS
SEARCHED (Int. Cl.5 )

A61N

The present search report has been drawn up for all claims

| Place of search | Date of completion of the search | Examiner |
|---|---|---|
| THE HAGUE | 02 OCTOBER 1992 | LEMERCIER D.L.L. |

EPO FORM 1503 03.82 (P0401)